# EUROPEAN PATENT SPECIFICATION

(11) **EP 3 123 172 B1**
(45) Date of publication and mention of the grant of the patent: **13.01.2021**
(21) Application number: 15717699.1
(22) Date of filing: 20.03.2015
(51) Int. Cl.: G01N 33/543, G01N 33/58, G01N 21/65, G01N 21/77

(54) **BIOASSAY SYSTEM AND METHOD FOR DETECTING ANALYTES IN BODY FLUIDS**
BIOASSAY-SYSTEM UND VERFAHREN ZUM NACHWEIS VON ANALYTEN IN KÖRPERFLÜSSIGKEITEN
SYSTÈME D'ESSAI BIOLOGIQUE ET PROCÉDÉ DE DÉTECTION D'ANALYTES DANS DES FLUIDES CORPORELS

(30) Priority: 24.03.2014 US 201461969371 P
(43) Date of publication of application: 01.02.2017
(73) Proprietor: Instrumentation Laboratory Company, Bedford, MA 01730 (US)
(72) Inventor: KERIMO, Josef, Bedford, Massachusetts 01730 (US); ZENG, Hansong, Bedford, Massachusetts 01730 (US); SCHARLACK, Ron, Bedford, Massachusetts 01730 (US); BLANKENSTEIN, Gert, Bedford, Massachusetts 01730 (US)
(74) Representative: Lock, Graham James
(86) International application number: PCT/US2015/021651
(87) International publication number: WO 2015/148290

(56) References cited:
- WO-A1-2013/095302
- WO-A1-2014/011118
- US-A1- 2004 191 765
- MURIANO ALEJANDRO ET AL: "High-sensitive nonlinear detection of steroids by resonant double grating waveguide structures-based immunosensors", INTEGRATED OPTICS: DEVICES, MATERIALS, AND TECHNOLOGIES XV, SPIE, 1000 20TH ST. BELLINGHAM WA 98225-6705 USA, vol. 7941, no. 1, 10 February 2011 (2011-02-10), pages 1-7, XP060020591, DOI: 10.1117/12.876456
- QIUQIANG ZHAN ET AL: "Multi-photon evanescent wave (MPEW) excited lanthanide-doped upconverting nanoparticles (UCNPs) for fast single particles tracking and live cell membrane imaging", COMMUNICATIONS AND PHOTONICS CONFERENCE (ACP), 2012 ASIA, IEEE, 7 November 2012 (2012-11-07), pages 1-3, XP032389716, ISBN: 978-1-4673-6274-0
- HENNA PÄKKILÄ ET AL: "Quantitative Multianalyte Microarray Immunoassay Utilizing Upconverting Phosphor Technology", ANALYTICAL CHEMISTRY, vol. 84, no. 20, 16 October 2012 (2012-10-16), pages 8628-8634, XP055112265, ISSN: 0003-2700, DOI: 10.1021/ac301719p

## Description

### TECHNICAL FIELD

The present invention relates to a bioassay system and a method for sensing analytes in a body fluid. More particularly, the present invention relates to a bioassay apparatus, for example, an immunoassay apparatus, comprising a resonance grating structure, and a method for detecting analytes in a body fluid using upconverting nanoparticles and the resonance grating structure.

### BACKGROUND

Traditionally, fluorophores are used as labels in bioassay apparatuses. However, fluorophores suffer photobleaching as time elapses.

Muriano Alejandra et al., Integrated Optics : Devices, Materials, and Technologies XV, USA, vol. 7941, no. 1, 10 February 2011, pages 1-7, discloses a bioassay system for the detection of steroids comprising a waveguide comprising a resonance grating structure having a grating surface and antibodies covalently attached to the grating surface, wherein the analyte methylboldenone is detected by two photon fluorescence by using a rhodamine B labeled boldenone

US2004/191765 A1 discloses a biosensor comprising a resonance waveguide grating structure defining an enhancement region with antibodies coupled to said enhancement region.

Henna Päkkilä et al., Analytical Chemistry, vol. 84, no. 20, 16 October 2012 (2012-10-16), pages 8628-8634, discloses an immunoassay utilizing luminescent upconverting single-crystal nanoparticles as reporters.

Various attempts have been made to use upconverting nanoparticles (i.e., particles having a diameter of between 1 and 100 nanometers and emitting light at a wavelength shorter than that of the excitation) as labels in bioassay apparatuses, because upconversing nanoparticles do not photobleach. However, the use of upconverting nanoparticles triggers other problems. For example, the emission from upconverting nanoparticles is rather weak due to the low quantum efficiency of upconverting nanoparticles and relatively high light levels are required for their excitation (e.g., 100 mW of 980 nm focused light). The upconverted emission is weak because the quantum efficiency (QE) of upconversion is typically less than 0.3%. In comparison, conventional fluorescence labels are much brighter because their QE can be greater than 20%. One approach for improving the upconversion emission is to chemically modify the nanoparticle surface but the success has been limited.

Upconverting nanoparticles are promising fluorophores and/or labels in bioassays, because they provide an almost background-free detection system with substantially no photobleaching effects. "Upconverting" as used herein means the emission of light at a wavelength shorter than that of the excitation light. For example, the nanoparticles may absorb multiple near-infrared (NIR) photons (e.g., two or three photons at about 980 nm) and then emit green light (about 510 nm) or red light (about 650 nm) in the visible region. The "upconversion" process (i.e., emission at a wavelength shorter than that of the excitation) rarely occurs in biological samples. It can be observed with conventional continous wave (CW) light sources (e.g., femto second pulsed NIR lasers with high peak power, causing high autofluorescence, are not required). Accordingly, a probing light beam would only trigger emission from the upconverting nanoparticles, and would almost never trigger emission from the biological samples. Therefore, the use of upconverting nanoparticles can lead to almost background-free detection when NIR CW light is used for the excitation and the blue-shifted emission is detected.

In bioassay applications, such background-free detection can lead to improvements in sensing capabilities. Typically, the background level determines the lower limit of detection in the assay and when it is too high the measurement precision suffers. In particular, UV light sources are commonly used in bioassays, which lead to high background from autofluorescence and scattering from the sample, and in the analysis of body fluid samples such as whole blood cannot be analyzed.

Accordingly, there is a need to develop a new bioassay apparatus that could solve the problem of photobleaching, reduce background and light scattering that is characteristic of conventional light sources, e.g., UV light, and enhance emission from target analytes. The use of upconverting nanoparticles as labels to overcome the problem of weak emission, fluorophore bleaching, and background autofluorescence from the sample is described in greater detail below.One objective of the present invention is to enhance the emission from upconverting nanoparticles in conjunction with resonant grating structures to achieve a high sensitivity biosensing platform for detecting analytes, such as, but not limited to proteins, pathogens, and electrolytes in body fluids. The enhanced emission of upconverting nanoparticles is important since the lower background signal and higher sensitivity of upconverting nanoparticles over traditional fluorescent dyes, quantum dots, or other fluorescent labels lead to superior performance in measurement accuracy in a bioassay, for example but not limited to immunoassay applications.

### SUMMARY OF THE INVENTION

The present invention is as defined in the appendant claims.

In one aspect, the present invention provides a bioassay system for detecting target analytes in body fluids, e.g., blood, serum, plasma, synovial fluid, cerebrospinal fluid, and urine, comprising: an upconverting nanoparticle; a waveguide comprising a resonance grating structure having a grating surface; a first antibody conjugated to the upconverting nanoparticle and directed to a first epitope in the target analyte; and a second antibody coupled to the grating surface and directed to a second epitope in the target analyte, wherein the second epitope is different from the first epitope and said second antibody is different from said first antibody; and wherein the resonance grating structure defines an enhancement region extending from said grating surface, said enhancement region configured to enhance excitation of the upconverting nanoparticle. The upconverting nanoparticle may comprise an optical property that absorbs infrared light and emits visible light in response to absorption of the infrared light.

In the system according to the present invention, the target analyte is bound to an anti-target analyte antibody conjugated to an upconverting nanoparticle, for example, NaYF₄:(Yb,Er,Tm), NaYbF₄:(Yb,Er,Tm), CaF₂:(Yb,Er), La₂O₃:(Yb,Er). The target analyte is also bound to a second anti-target analyte antibody (that differs from the first anti-target analyte antibody) that may be coupled to the surface of the resonance grating structure through a linking chemistry, for example, through strepavidin. Accordingly, the target analyte is "labeled" by a nanoparticle by the first antibody, and "captured" to the surface of the resonance grating structure by the second antibody. At the surface of the resonance grating structure, the target analyte is optically detected.

In one embodiment, the bioassay sensor further comprises a highly refractive layer (e.g., TiO₂ and Ta₂O₅) disposed on the surface of the resonance grating structure, the highly refractive layer having a refractive index of at least 1.5. The resonance grating structure may comprise a photonic crystal tuned to a predetermined resonance condition and an antibody coupled to the resonsance grating structure through a linkage chemistry.

In another aspect, the present invention is directed to an apparatus for detecting a target analyte, comprising a resonance waveguide grating structure, the resonance waveguide grating structure defining a grating surface and an enhancement region extending from said grating surface, the resonance waveguide grating structure comprising a plurality of capturing sites on the grating surface; a refractive layer disposed on the capturing sites; a first antibody directed to a first epitope in said target analyte and conjugated to a plurality of upconverting nanoparticles; and a second antibody, the second antibody different from the first antibody and directed to a second epitope in said target analyte, wherein the second epitope is different from the first epitope, and the second antibody is positioned at the grating structure and coupled to the capturing sites; a light source directed to said capturing sites, the light source configured for generating an optical signal of a first wavelength to exite the upconverting nanoparticles; and a light detector configured for sensing an optical response of a second wavelength from said capturing sites, wherein the second wavelength is shorter than the first wavelength.

In yet another aspect, the present invention is directed to an immunoassay kit for detecting a target analyte, comprising: a bioassy system having a resonance grating substrate and a second antibody coupled to a surface of the resonance grating substrate through a chemical linkage; and a composition of matter having a first antibody conjugated to an upconverting nanoparticle, said first antibody different from the second antibody, wherein the first and second antibodies are directed to different epitopes of the same target analyte.

In yet another aspect, the present invention is directed to a method for detecting a target analyte in a body fluid, comprising: providing a resonance grating structure comprising a plurality of capturing sites; the resonance grating structure having one or more target analytes captured at the capturing sites through a second antibody directed to a second epitope of the target analyte, a first antibody of the target analyte being conjugated to an upconverting nanoparticle, wherein the first epitope is different from the second epitope; applying an optical illumination to the resonance grating structure; and detecting optical responses from the capturing sites, said optical response from one of the capturing sites being indicative of the presence of the target analyte at that capturing site.

A resonance grating structure having a plurality of capturing sites is provided. An optical illumination is provided to the resonance grating structure which has one or more target analytes coupled to an upconverting particle captured via an antibody at the capturing site. Optical responses from the capturing sites are detected and are indicative of the presence of the target analyte at the corresponding capturing sites.

As used herein, the term coupled means at least two elements joined together directly or indirectly.

### BRIEF DESCRIPTION OF THE DRAWINGS

Figure 1A schematically illustrates a sectional view and Figure 1B illustrates a perspective view of a resonant waveguide having a grating structure for use in the bioassay system, apparatus, kit or method of the present invention.
Figure 2 schematically illustrates the electric field on the surface of a substrate under a resonance condition.
Figure 3 schematically illustrates a method for sensing analytes using a bioassay system, such as an immunoassay, in accordance with one embodiment of the present invention.
Figure 4 schematically illustrates a method for sensing analytes using a bioassay system, such as an immunoassay, in accordance with another embodiment of the present invention.

### DESCRIPTION

One approach of increasing bioassay sensitivity is to increase the light level of the illumination by using a polymer-based grating substrate to boost the emission, but this can lead to certain problems, such as high background and increased sample temperature and sample damage. For example, in such a bioassay system, the polymer-based grating substrate begins to present excessive amount of background due to autofluorescence. This interference from the background is not suitable for high sensitivity biosensing applications, for example, immunoassays. High quality and purity quartz substrates have been used to address this problem, but these materials have led to much higher material cost and more complicated manufacturing processes. Accordingly, the present invention employs upconverting nanoparticles that absorb infrared excitation in conjunction with resonant grating structures to achieve high sensitivity in a biosensing platform for detecting analytes.

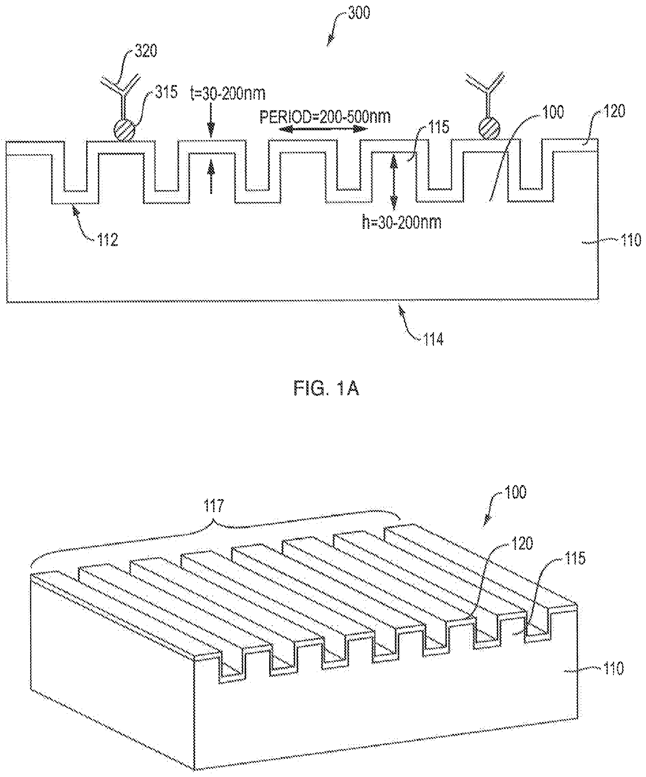

Referring to Figures 1A and 1B, Figure 1A illustrates a sectional view and Figure 1B illustrates a perspective view of a sensor 300 comprising a resonant waveguide 100 and a secondary antibody 320 directed to a target analyte, the secondary antibody being bound to the surface of the resonant waveguide 100 through a chemical linkage 315. The chemical linkage 315 may comprise a binding protein, such as streptavidin.

With continued reference to Figure 1A and 1B, resonant waveguide 100 comprises a substrate 110 having a grating structure 115 formed on a surface 112 of the substrate 110, and a refractive layer 120 formed on the surface 112 of grating structure 115. One or more additional refractive layers (not shown) may be formed on refractive layer 120.

With continued reference to Figure 1A, substrate 110 is made of an optically transparent material or a polymeric material (for example, but not limited to, polystyrene, ultraviolet curable polymer or glass), and grating structure 115 is formed to have a grating period of about 360 nm, a grating groove of about 50 nm in depth, and a duty cycle of about 36%. It is appreciated that the grating period ranges from about 200 nm to about 500 nm; the grating groove depth ranges from about 30 nm to about 300 nm; the thickness of refractive layer 120 (having a refractive index of greater than 1.5) ranges from about 30 nm to about 200 nm; and the duty cycle ranges from about 30% to about 50%. The substrate 110 is transparent and compatible with the near-infrared (NIR) excitation and visible light detection of emission from upconverting nanoparticles. The grating material is made from, for example, but not limited to, SiO₂, polymeric material such as polystyrene, silicone, thermoplastics, or glass such as fused silica and quartz.

Refractive layer 120 can be made of a high refractive material (e.g., TiO₂ with a refractive index of about 2.35, or Ta₂O₅ with a refractive index of about 2.09). It is appreciated that various forms and configurations of the grating are possible to produce a resonance mode, and can enhance the excitation of upconverting nanoparticles. For example, the grating may have more than one layer of thin coating of high refractive material, and may enhance the intensity of excitation light when nanoparticles are close to the surface 112 (in the range of about 1-150 nm, preferably in the range of about 1-2000 nm, more preferably below 300 nm). The resulting surface of grating structures 115 and refractive layer 120 behave much like a grating, but tuned to specific resonance modes and wavelengths where the enhancement occurs. At the resonance wavelength, the evanescent electrical field on or above the substrate surface 112 is very high (e.g., greater than 50-fold than without the grating) so surface labels such as upconverting nanoparticles can emit strongly. Although refractive layer 120 is shown and described in Figure 1A, it is to be understood that resonant waveguide 100 may still constitute a photonic crystal, without the presence of refractive layer 120.

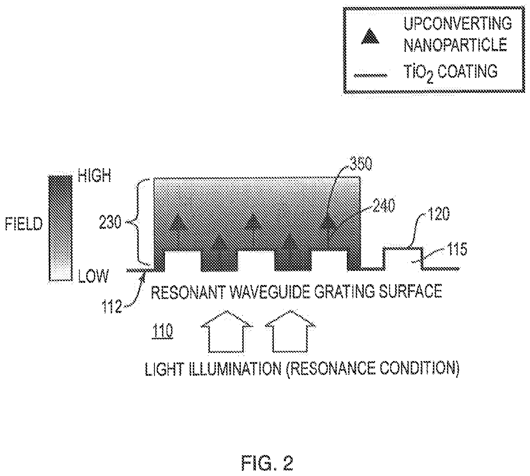

Figure 2 schematically illustrates the electric field on the surface of a substrate 110 under a resonance condition. As shown in Figure 2, a light beam 210 impinges from a surface 114 (see Figure 1A) of substrate 110 opposite to the surface 112. A plurality of upconverting nanoparticles 350 are bound to the surface 112 of grating structure 115, on which a refractive layer 120 is formed. The upconverting nanoparticles 350 may be conjugated to an antibody (not shown) that is directed to a target analyte (not shown). The target analyte is bound to the nanoparticle conjugated antibody and captured at the surface 112 by a second antibody (not shown) directed to the target analyte. The second antibody is bound to the surface 112 through a linkage chemistry 240 (for example, but not limited to, streptavidin, surface couplings via reactive functional groups such as amino, hydroxyl, thiol, and carboxyl groups, modified DNA probes, and peptides).

Referring to Figures 1A, 1B, and 2, the wavelength of light beam 210 directed to surface 114 of substrate 110 matches a resonance condition of the grating structure 115 that constitutes a photonic crystal. As a result, the intensity of light beam 210 is greatly enhanced at an enhancement region 230 over a textured surface 117 of substrate 110. In this embodiment, textured surface 117 is formed of grating structure 115 (parallel ridges and valleys), as shown in Figure 1B. It is appreciated that textured surface 117 may be formed of an array of protrusions (e.g., pillars and rods), or an array of recesses (e.g., circular recesses, rectangular recesses, and hexagonal recesses). In an ideal situation, the enhancement of the light beam intensity can be as high as 1500-fold with respect to the intensity of light beam 210 without using a photonic crystal. The enhancement may be less than this and may reach around 50-fold.

Other materials useful for enhancing the emission are, for example, plasmonic surface structures made from metallic films. In one example, the emission of upconverting nanoparticles is enhanced almost 300-fold, using plasmonic nanoantenna (gold dots on pillar structures). The advantage of this is that the upconverting nanoparticles/labels are more robust and do not photobleach and last a long time compared to conventional fluorophores (e.g., several hours versus a few minutes for conventional fluorophores). However, the plasmonic structures are in general more difficult to manufacture and may have several drawbacks, such as strong light absorption that can cause heating effects. A transparent dielectric material avoids this problem.

A high sensitivity system described herein for biomedical assay sensing applications includes two components. For example, a first component is a sensor comprising a resonance grating structure made from a transparent dielectric material, such as a photonic crystal waveguide, and a secondary antibody directed to a target analyte that is bound to the surface of the resonance grating structure. A second component may comprise an upconverting nanoparticle bound to a primary antibody directed to the same target analyte as the secondary antibody. Conjugates other than an upconverting nanoparticle are also contemplated, for example, a conventional fluorophore or a downconverting nanoparticle. The system is designed for high sensitivity assay applications to provide low cost and ease of mass production and minimal background signal.

With continued reference to Figure 2, the system of the present invention has improved sensitivity with the enhanced fields at enhancement region 230. The upconverting particles 350 are located in the enhancement region 230 of the resonance grating structure 100 which extends for more than a hundred nanometers from surface 112 of substrate 110. It is appreciated that enhancement region 230 has a brightline boundary at surface 112, but a rather blurry boundary as the electric field which gradually diminishes away from surface 112. The blurry boundary may be defined as a contour line where the electric field is half of the strongest electric field proximate surface 112. The emission may be enhanced by about 50-fold or more, to improve the assay sensitivity.

The present invention has numerous advantages over conventional immunoassay systems. First, the present invention does not suffer from intensive background signal emanating from autofluorescence. Grating structure 115 may have a grating period, a grating groove depth, a duty cycle, such that the resonance of grating 115 is tuned to the 980 nm absorption peak of the upconverting nanoparticles 350 and to normal incidence of the illuminating light source 210. In this case, there is substantially no background from the substrate 110 because the 980 nm light does not cause autofluorescence from grating 115 or from the body fluid sample being analyzed.

The bioassay system according to the invention has additional advantages over conventional bioassay systems, such as immunoassay systems. For example, the system does not suffer from photobleach of fluorophores. Upconverting nanoparticles 350 are known to be very stable and do not photobleach and can better handle the high electric fields proximate surface. Conventional fluorophores are less desirable and photobleach quickly.

Additionally, the system of the present invention has enhanced localized surface emission. Upconverting nanoparticles 350 have nonlinear absorption dependence with respect to the illumination intensity. Because of this nonlinear property, only bound nanoparticles, or those that are close to the surface, are excited selectively. As a result, there is little emission from the surrounding media.

Further, the system of the present invention is applicable in homogeneous assay applications. Because of the enhanced surface-emission of bound nanoparticles, the assay washing step can be eliminated leading to a much simpler system design.

Moreover, the system of the present invention does not require focused light illumination. In the invention disclosed herein, a laser (or a coherent light source) is not needed since the enhancement effect from the resonance grating increases the intensity without the need for focusing and high power of lasers. Low-cost incoherent sources, for example, but not limited to, LED, gas discharge lamps, and high-intensity discharge lamps can be used instead of a laser.

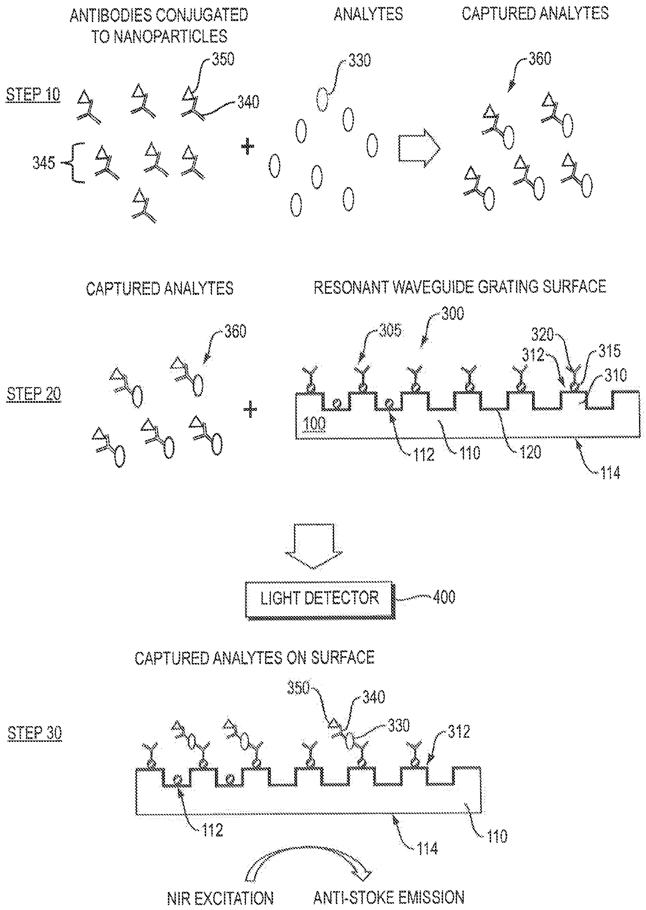

Figure 3 schematically illustrates a method for detecting analytes using a bioassay apparatus in accordance with the present invention. In Step 10, a composition of matter 345 comprising a nanoparticle 350 conjugated to a first antibody 340 targeted to a target analyte 330 is mixed with a body fluid such as blood, serum, plasma, synovial fluid, cerebrospinal fluid , or urine in which the target analyte 330 is suspected to be present. The composition of matter 345 may be formed by mixing nanoparticles 350 and first antibodies 340 directed to the target analyte in an organic or inorganic solvent for coupling nanoparticles to antibodies. Alternatively, the nanoparticle labeled antibodies may be a powder form that is mixed in the fluid containing the suspected analytes 330 to be detected.

In the next step, Step 20, nanoparticle labeled antibodies 345 bound to analytes 330 are applied to a bioassay sensor 300 having a resonant waveguide grating surface 112. Bioassay sensor 300 may comprise a substrate 110 having a grating structure 310, a refractive layer 120 formed on the surface of grating structure 310, and second antibodies 320 directed to the target analyte 330 coupled and immobilized to a surface 312 of grating structure 310. Each grating period of grating structure 310 may comprise an anti-target antibody coupled therewith to constitute a target analyte capturing site 305. Second antibodies 320 may be immobilized on grating structure 310 through linkage chemistry 315 (e.g., streptavidin) to capture target analytes 330 coupled to, for example, a nanoparticle selected from the group consisting of NaYF4: Yb-Er, CaF2:Yb,Er, NaYbF4:Ho,Tm, Er from the body fluid of a patient being analyzed. Captured analytes 360 are bound to second antibodies 320 that are directed to the same target analytes 330 and are coupled to the surface 312 of grating structure 300. Bioassay sensor 300 may be washed by pure water to remove uncaptured analytes from substrate 110. Bioassay sensor 300 is now ready for optical examination.

In Step 30, light, e.g., a near-infrared (NIR) light beam having a wavelength of about 980 nm is applied to a surface 114 of substrate 110 that is on the side of the substrate 110 of grating structure 300 opposite to the surface 312 which may include refractive layer 120. Because the NIR light beam is chosen to match the resonance condition of grating structure 310, the upconverting nanoparticles 350 conjugated to the first antibody 340 directed to the target analyte 330 and bound to the grating surface 312 through the second antibody 320 also directed to the target analyte 330 are excited by an enhanced NIR excitation. Depending on the optical property of upconverting nanoparticles 350, a light beam (such as visible light) is emitted in response to the enhanced NIR excitation. The presence and/or absence of analytes 330 on capturing sites 350 is determined using a light detector 400.

An upconverting nanoparticle 350 may be conjugated to an antibody 340 in a "sandwich" assay to detect the captured target analyte 330 bound to a second antibody directed to the target analyte 330 at capturing sites 305 on the surface of a resonant grating structure 310. The resonance of grating structure 310 is tuned to the peak of their absorption peak to have the largest enhancement effect and to improve the assay sensitivity.

The bioassay sensor may be used in a "blocking assay" application where binding of particles to the surface of the resonant grating structure 310 leads to "detuning" of the resonance mode and reduction of the enhancement effect. The surface binding events result in change of the refractive index of refractive layer 120 and are enough to detune the resonance away from the illumination wavelength. For example, a laser with a very narrow wavelength bandwidth and a grating with a high quality factor resonance structure, or a very narrow and sharp resonance peak, is a very sensitive arrangement for detecting changes to the refractive index at the surface. As the resonance gets detuned away from the laser wavelength, a sharp drop in the enhanced emission of the upconverting nanoparticles may be observed.

The sensing system of the present invention may take advantage of the relatively narrow enhancement region where the enhancement takes place and is useful to detect binding kinetics and follow changes in the quantity of target analyte over time. Not to be bound by theory but it is believed that the emission from upconverting particles captured at the surface of the grating structure is confined to less than a 100 nm region proximate the grating surface. Any upconverting nanoparticle labels outside the 100 nm region are not excited and consequently have much less emission than nanoparticles that are positioned closer to the surface of the grating structure and within the 100 nm region.

For example, the grating surface 312 may first be pretreated with labels (such as fluorophores or nanoparticles) by predefined linkage elements (such as thrombin) that are susceptible to cleavage by the action of a target protein. When exposed to thrombin, the labels are cleaved at the linkage element and diffuse away from the enhancement region. Thus, cleavage of the linkage element frees the label away from the surface in the 100 nm region above the grating structure and the label cannot be detected. Decreased emission allows for quantitative determination of a target protein, such as thrombin. In another example, the labels can specifically bind to the surface 312 and increase the signal indicating the binding events.

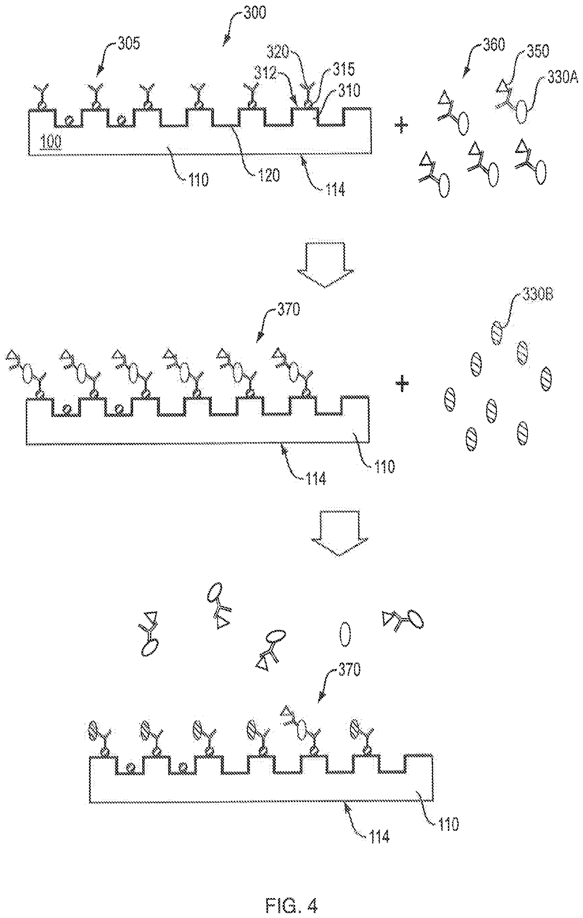

As shown in Figure 4, the bioassay system is configured in a competitive heterogeneous immunoassay mode where a target analyte 330A, for example, is bound to an upconverting nanoparticle to form a nanoparticle labeled conjugated analyte 360. The target analyte 330B in the patient's body fluid sample is unlabeled. The nanoparticle labeled conjugated analyte 360 is then applied to bioassay sensor 300, such that capturing sites 305 are bound with a conjugated analyte 360. Then, the patient's body fluid including unlabeled analytes 330B undergoing analysis is applied to the resultant "labeled" bioassay sensor 300. The patient's unlabeled target analyte 330B competes for binding sites 305 on the grating surface-coupled anti-target analyte antibody 320 with the nanoparticle labeled target analyte 360. Upon introduction of the patient's unlabeled body fluid target analyte 330B, the labeled target analytes 360 are released from the grating surface 312 and are free to diffuse into the solution away from the enhancement region 230 (see Figure 2). Detectable light emission arising from the nanoparticle labeled target analyte 360 decreases in the presence of patient unlabeled analyte 330B. This configuration is commonly used in drug analysis and in clinical biochemistry of hormones and proteins. In this mode, unlabeled analyte 330B in the patient sample competes with nanoparticle-labeled analyte 360 at the enhancing surface 312. The unbound analyte is washed away, and the remaining labeled analyte 370 bound to the grating surface is measured. A decrease in emission of the labeled bound analyte 360 is proportional to the amount of target analyte in the patient's body fluid sample.

Although embodiments of the present disclosure have been described in detail, it is to be understood that these embodiments are provided for exemplary and illustrative purposes only.

## Claims

1. A bioassay system (300) for detecting a target analyte (330), comprising:
an upconverting nanoparticle (350);
a waveguide comprising a resonance grating structure (200) having a grating surface (312);
a first antibody (340) conjugated to the upconverting nanoparticle (350) and directed to a first epitope in the target analyte (330); and
a second antibody (320) coupled to the grating surface (312) and directed to a second epitope in the target analyte (330), wherein the second epitope is different from the first epitope and said second antibody (320) is different from said first antibody (340); and
wherein the resonance grating structure (100) defines an enhancement region (230) extending from said grating surface (312), said enhancement region (230) configured to enhance excitation of the upconverting nanoparticle (350).

2. The system of claim 1, wherein the upconverting nanoparticle (350) comprises an optical property that absorbs infrared light and emits visible light in response to absorption of the infrared light.

3. The system of claim 1, wherein the upconverting nanoparticle (350) is coupled to the grating surface (312) through the target analyte (330) to be detected.

4. The system of claim 3, wherein the upconverting nanoparticle (350) is conjugated to the target analyte (330) through the first antibody (340), and the target analyte (330) is coupled to the surface (312) of the resonance grating structure (100) through the second antibody (320).

5. The system of claim 4, wherein the second antibody (320) is coupled to the surface (312) of the resonance grating structure (100) through a chemical linkage (315).

6. The system of claim 1, further comprising a refractive layer (120) on the grating surface (312) having a refractive index of at least 1.5.

7. The system of claim 1, wherein the resonance grating structure (100) comprises a photonic crystal tuned to a predetermined resonance condition.

8. The system of claim 1, comprising:
a plurality of upconverting nanoparticles (350) coupled to the first antibody (340);
wherein the first antibody specific to the target analyte (330) is conjugated to upconverting nanoparticles; and
the second antibody (320) specific to the target analyte (330) is coupled to a surface of the resonance grating structure (100) through a chemical linkage (315).

9. The system of claim 8, further comprising a refractive layer on the surface of the resonance grating structure (100), the refractive layer having a refractive index of a least 1.5.

10. The system of claim 9, wherein the resonance grating structure (100) comprises a photonic crystal selected from the group consisting of replicated gratings, holographic photonic crystal, and porous silicon photonic crystal tuned to a predetermined resonance condition.

11. A method for detecting a target analyte (330) in a body fluid, comprising:
providing a resonance grating structure (100) comprising a plurality of capturing sites (350);
the resonance grating structure (100) having one or more target analytes captured at the capturing sites (350) through a second antibody (320) directed to a second epitope of the target analyte (330), a first antibody of the target analyte being conjugated to an upconverting nanoparticle, wherein the first epitope is different from the second epitope;
applying an optical illumination to the resonance grating structure (100); and
detecting optical responses from the capturing sites (350), said optical response from one of the capturing sites (350) being indicative of the presence of the target analyte (330) at that capturing site (350).

12. An apparatus for detecting a target analyte (330), comprising:
a resonance waveguide grating structure (100), the resonance waveguide grating structure (100) defining a grating surface (312) and an enhancement region (230) extending from said grating surface (312), the resonance waveguide grating structure comprising a plurality of capturing sites (350) on the grating surface (312);
a refractive layer (120) disposed on the capturing sites (350);
a first antibody (340) directed to a first epitope in said target analyte (330) and conjugated to a plurality of upconverting nanoparticles (350); and
a second antibody (320), the second antibody different from the first antibody (340) and directed to a second epitope in said target analyte, wherein the second epitope is different from the first epitope, and the second antibody (320) is positioned at the grating structure (100) and coupled to the capturing sites (350);
a light source (210) directed to said capturing sites, the light source (210) configured for generating an optical signal of a first wavelength to excite the upconverting nanoparticles (350); and
a light detector (400) configured for sensing an optical response of a second wavelength from said capturing sites, wherein the second wavelength is shorter than the first wavelength.

13. The apparatus of claim 12, wherein i) the first wavelength matches a resonance condition of the waveguide grating structure (100), or ii) the resonance waveguide grating structure (100) defines an enhancement region (230) that extends from a surface of the capturing sites (350) and along a direction normal to the surface for a predetermined distance, or iii) the second antibody (320) is respectively coupled to the capturing sites (350) through a chemical linkage (315).

14. An immunoassay kit for detecting a target analyte (330), comprising:
a bioassay system (300) having a resonance grating substrate and a second antibody coupled to a surface of the resonance grating substrate through a chemical linkage; and
a composition of matter (345) having a first antibody conjugated to an upconverting nanoparticle, said first antibody (340) different from the second antibody (320), wherein the first and second antibodies are directed to different epitopes of the same target analyte.

## Patentansprüche

1. Bioassaysystem (300) zum Erfassen eines Zielanalyten (330), welches umfasst:
einen Hochkonversions-Nanopartikel (350);
einen Wellenleiter, der einen Resonanzgitteraufbau (200) mit einer Gitteroberfläche (312) umfasst;
einen ersten Antikörper (340), der mit dem Hochkonversions-Nanopartikel (350) konjugiert und auf ein erstes Epitop in dem Zielanalyten (330) gerichtet ist; und
einen zweiten Antikörper (320), der mit der Gitteroberfläche (312) gekoppelt und auf ein zweites Epitop in dem Zielanalyten (330) gerichtet ist, worin das zweite Epitop von dem ersten Epitop verschieden und der zweite Antikörper (320) von dem ersten Antikörper (340) verschieden ist; und
worin der Resonanzgitteraufbau (100) einen Verstärkungsbereich (230) definiert, der sich von der Gitteroberfläche (312) erstreckt, worin der Verstärkungsbereich (230) ausgestaltet ist, eine Anregung des Hochkonversions-Nanopartikels (350) zu verstärken.

2. System nach Anspruch 1, worin der Hochkonversions-Nanopartikel (350) eine optische Eigenschaft umfasst, die Infrarotlicht absorbiert und als Reaktion auf die Absorption des Infrarotlichtes sichtbares Licht emittiert.

3. System nach Anspruch 1, worin der Hochkonversions-Nanopartikel (350) durch den zu erfassenden Zielanalyten (330) mit der Gitteroberfläche (312) gekoppelt ist.

4. System nach Anspruch 3, worin der Hochkonversions-Nanopartikel (350) mit dem Zielanalyten (330) durch den ersten Antikörper (340) konjugiert ist, und der Zielanalyt (330) durch den zweiten Antikörper (320) mit der Oberfläche (312) des Resonanzgitteraufbaus (100) gekoppelt ist.

5. System nach Anspruch 4, worin der zweite Antikörper (320) durch eine chemische Bindung (315) mit der Oberfläche (312) des Resonanzgitteraufbaus (100) gekoppelt ist.

6. System nach Anspruch 1, welches weiter auf der Gitteroberfläche (312) eine Refraktärschicht (120) mit einem Brechungsindex von mindestens 1,5 umfasst.

7. System nach Anspruch 1, worin der Resonanzgitteraufbau (100) einen photonischen Kristall umfasst, der auf eine bestimmte Resonanzbedingung eingestellt ist.

8. System nach Anspruch 1, welches umfasst:
mehrere Hochkonversions-Nanopartikel (350), die mit dem ersten Antikörper (340) gekoppelt sind;
worin der erste für den Zielanalyten (330) spezifische Antikörper mit Hochkonversions-Nanopartikeln konjugiert ist; und
der zweite für den Zielanalyten (330) spezifische Antikörper (320) durch eine chemische Bindung (315) der mit einer Oberfläche des Resonanzgitteraufbaus (100) gekoppelt ist.

9. System nach Anspruch 8, welches weiter eine Refraktärschicht auf der Oberfläche des Resonanzgitteraufbaus (100) umfasst, worin die Refraktärschicht einen Brechungsindex von mindestens 1,5 aufweist.

10. System nach Anspruch 9, worin der Resonanzgitteraufbau (100) einen photonischen Kristall umfasst, ausgewählt aus der Gruppe bestehend aus nachgebildeten Gittern, einem holografischen photonischen Kristall, und porösem photonischem Siliciumkristall, der auf eine bestimmte Resonanzbedingung eingestellt ist.

11. Verfahren zum Erfassen eines Zielanalyten (330) in einer Körperflüssigkeit, welches umfasst:
Bereitstellen eines Resonanzgitteraufbaus (100), der mehrere Bindestellen (350) umfasst;
worin der Resonanzgitteraufbau (100) eine oder mehrere Zielanalyten aufweist, die an den Bindestellen (350) durch einen zweiten Antikörper (320) erfasst werden, der an ein zweites Epitop des Zielanalyten (330) gerichtet ist, worin ein erster Antikörper des Zielanalyten mit einem Hochkonversions-Nanopartikel konjugiert ist, worin das erste Epitop von dem zweiten Epitop verschieden ist;
Anwenden einer optischen Illumination an den Resonanzgitteraufbau (100); und Erfassen optischer Antworten von den Bindestellen (350), wobei die optische Antwort von einer der Bindestellen (350) das Vorliegen des Zielanalyten (330) an der Bindestelle (350) anzeigt.

12. Vorrichtung zum Erfassen eines Zielanalyten (330), welche umfasst:
einen Resonanz-Wellenleiter-Gitteraufbau (100), worin der Resonanz-Wellenleiter-Gitteraufbau (100) eine Gitteroberfläche (312) und einen Verstärkungsbereich (230) definiert, der sich von der Gitteroberfläche (312) erstreckt, worin der Resonanz-Wellenleiter-Gitteraufbau mehrere Bindestellen (350) auf der Gitteroberfläche (312) umfasst;
eine Refraktärschicht (120), die auf den Bindestellen (350) angeordnet ist;
einen ersten Antikörper (340), der auf ein erstes Epitop in dem Zielanalyten (330) gerichtet und mit mehreren Hochkonversions-Nanopartikeln (350) konjugiert ist; und
einen zweiten Antikörper (320), worin der zweite Antikörper von dem ersten Antikörper (340) verschieden und an ein zweites Epitop in dem Zielanalyten gerichtet ist, worin das zweite Epitop von dem ersten Epitop verschieden ist, und worin der zweite Antikörper (320) an dem Gitteraufbau (100) angeordnet und mit den Bindestellen (350) gekoppelt ist;
eine Lichtquelle (210), die auf die Bindestellen gerichtet ist, worin die Lichtquelle (210) ausgestaltet ist, ein optisches Signal einer ersten Wellenlänge zu erzeugen, um die Hochkonversions-Nanopartikel (350) anzuregen; und
einen Lichtdetektor (400), der ausgestaltet ist, eine optische Antwort einer zweiten Wellenlänge von den Bindestellen zu erfassen, wobei die zweite Wellenlänge kürzer ist als die erste Wellenlänge.

13. Vorrichtung nach Anspruch 12, worin i) die erste Wellenlänge einer Resonanzbedingung des Wellenleiter-Gitteraufbaus (100) entspricht, oder ii) der Resonanz-Wellenleiter-Gitteraufbau (100) einen Verstärkungsbereich (230) definiert, der sich um eine bestimmte Entfernung von einer Oberfläche der Bindestellen (350) und entlang einer senkrechten Richtung zu der Oberfläche erstreckt, oder iii) der zweite Antikörper (320) durch eine chemische Bindung (315) mit den Bindestellen (350) entsprechend gekoppelt ist.

14. Immunoassay-Kit zum Erfassen eines Zielanalyten (330), welches umfasst:
ein Bioassaysystem (300) mit einem Resonanzgittersubstrat und einem zweiten Antikörper, der durch eine chemische Bindung mit einer Oberfläche des Resonanzgittersubstrates gekoppelt ist; und
eine Zusammensetzung (345) mit einem ersten Antikörper, der mit einem Hochkonversions-Nanopartikel konjugiert ist, worin der erste Antikörper (340) von dem zweiten Antikörper (320) verschieden ist, worin der erste und der zweite Antikörper an verschiedene Epitope des gleichen Zielanalyten gerichtet sind.

## Revendications

1. Système d'essai biologique (300) pour détecter un analyte cible (330), comprenant :
une nanoparticule à conversion ascendante (350) ;
un guide d'ondes comprenant une structure de réseau de résonance (200) ayant une surface de réseau (312) ;
un premier anticorps (340) conjugué à la nanoparticule à conversion ascendante (350) et dirigé contre un premier épitope dans l'analyte cible (330) ; et
un second anticorps (320) couplé à la surface de réseau (312) et dirigé contre un second épitope dans l'analyte cible (330), dans lequel le second épitope est différent du premier épitope et ledit second anticorps (320) est différent dudit premier anticorps (340) ; et
dans lequel la structure de réseau de résonance (100) définit une région d'augmentation (230) s'étendant de ladite surface de réseau (312), ladite région d'augmentation (230) configurée pour augmenter l'excitation de la nanoparticule à conversion ascendante (350).

2. Système selon la revendication 1, dans lequel la nanoparticule à conversion ascendante (350) comprend une propriété optique qui absorbe la lumière infrarouge et émet de la lumière visible en réponse à l'absorption de la lumière infrarouge.

3. Système selon la revendication 1, dans lequel la nanoparticule à conversion ascendante (350) est couplée à la surface de réseau (312) par l'intermédiaire de l'analyte cible (330) à détecter.

4. Système selon la revendication 3, dans lequel la nanoparticule à conversion ascendante (350) est conjuguée à l'analyte cible (330) par l'intermédiaire du premier anticorps (340), et l'analyte cible (330) est couplé à la surface (312) de la structure de réseau de résonance (100) par l'intermédiaire du second anticorps (320) .

5. Système selon la revendication 4, dans lequel le second anticorps (320) est couplé à la surface (312) de la structure de réseau de résonance (100) par l'intermédiaire d'une liaison chimique (315).

6. Système selon la revendication 1, comprenant en outre une couche réfractive (120) sur la surface de réseau (312) ayant un indice de réfraction d'au moins 1,5.

7. Système selon la revendication 1, dans lequel la structure de réseau de résonance (100) comprend un cristal photonique accordé à un état de résonance prédéterminé.

8. Système selon la revendication 1, comprenant :
une pluralité de nanoparticules à conversion ascendante (350) couplées au premier anticorps (340) ;
dans lequel le premier anticorps spécifique de l'analyte cible (330) est conjugué à des nanoparticules à conversion ascendante ; et
le second anticorps (320) spécifique de l'analyte cible (330) est couplé à une surface de la structure de réseau de résonance (100) par l'intermédiaire d'une liaison chimique (315) .

9. Système selon la revendication 8, comprenant en outre une couche de réfraction sur la surface de la structure de réseau de résonance (100), la couche de réfraction ayant un indice d'au moins 1,5.

10. Système selon la revendication 9, dans lequel la structure de réseau de résonance (100) comprend un cristal photonique choisi dans le groupe consistant en des réseaux répliqués, un cristal photonique holographique et un cristal photonique de silicium poreux accordé à un état de résonance prédéterminé.

11. Procédé de détection d'un analyte cible (330) dans un fluide corporel, comprenant :
fournir une structure de réseau de résonance (100) comprenant une pluralité de sites de capture (350) ;
la structure de réseau de résonance (100) ayant un ou plusieurs analytes cibles capturés au niveau de sites de capture (350) par l'intermédiaire d'un second anticorps (320) dirigé contre un second épitope de l'analyte cible (330), un premier anticorps de l'analyte cible étant conjugué à une nanoparticule à conversion ascendante, dans lequel le premier épitope est différent du second épitope ;
appliquer un éclairage optique à la structure de réseau de résonance (100) ; et
détecter les réponses optiques des sites de capture (350), ladite réponse optique de l'un des sites de capture (350) étant indicatrice de la présence de l'analyte cible (330) au niveau de ce site de capture (350).

12. Appareil pour détecter un analyte cible (330), comprenant :
une structure de réseau de guide d'ondes de résonance (100), la structre de réseau de guide d'ondes de résonance (100) définissant une surface de réseau (312) et une région d'augmentation (230) qui s'étend de ladite surface de réseau (312), la structure de réseau de guide d'ondes de résonance comprenant une pluralité de sites de capture (350) sur la surface de réseau (312) ;
une couche réfractive (120) disposée sur les sites de capture (350) ;
un premier anticorps (340) dirigé contre un premier épitope dans ledit analyte cible (330) et conjugué à une pluralité de nanoparticules à conversion ascendante (350) ; et
un second anticorps (320), le second anticorps différent du premier anticorps (340) et dirigé contre un second épitope dans ledit analyte cible, dans lequel le second épitope est différent du premier épitope, et le second anticorps (320) est positionné au niveau de la structure de réseau (100) et couplé aux sites de capture (350) ;
une source de lumière (210) dirigée vers lesdits sites de capture, la source de lumière (210) configurée pour générer un signal optique d'une première longueur d'onde pour exciter les nanoparticules à conversion ascendante (350) ; et
un détecteur de lumière (400) configuré pour détecter une réponse optique d'une seconde longueur d'onde à partir desdits sites de capture, dans lequel la seconde longueur d'onde est plus courte que la première longueur d'onde.

13. Appareil selon la revendication 12, dans lequel i) la première longueur d'onde correspond à un état de résonance de la structure de réseau de guide d'ondes (100), ou ii) la structure de réseau de guide d'ondes de résonance (100) définit une région d'augmentation (230) qui s'étend d'une surface des sites de capture (350) et le long d'une direction normale par rapport à la surface sur une distance prédéterminée, ou iii) le second anticorps (320) est respectivement couplé aux sites de capture (350) par l'intermédiaire d'une liaison chimique (315).

14. Kit d'immunoessai pour détecter un anaiyte cible (330), comprenant :
un système d'essai biologique (300) ayant un substrat de réseau de résonance et un second anticorps couplé à une surface du substrat de réseau de résonance par l'intermédiaire d'une liaison chimique ; et
une composition de matière (345) ayant un premier anticorps conjugué à une nanoparticule à conversion ascendante, ledit premier anticorps (340) différent du second anticorps (320), dans lequel les premier et second anticorps sont dirigés contre différents épitopes du même analyte cible.
